(19) Europäisches Patentamt
European Patent Office
Office européen des brevets

(11) EP 1 000 543 A1

(12) EUROPEAN PATENT APPLICATION

(43) Date of publication:
17.05.2000 Bulletin 2000/20

(51) Int. Cl.[7]: **A01N 35/02**, C12N 15/63

(21) Application number: 99203496.7

(22) Date of filing: 29.12.1995

(84) Designated Contracting States:
AT BE CH DE DK ES FR GB GR IE IT LI LU MC NL PT SE

(30) Priority: 30.12.1994 US 367082
07.06.1995 US 485035

(62) Document number(s) of the earlier application(s) in accordance with Art. 76 EPC:
95944684.0 / 0 800 345

(71) Applicant: PROGUARD, INC.
Suisun City, CA 94585 (US)

(72) Inventors:
- Emerson, Ralph W.
Davis, California 95616 (US)
- Crandall, Bradford G., Jr.
Davis, California 95616 (US)

(74) Representative:
Ottevangers, Sietse Ulbe
Vereenigde Octrooibureaux,
Postbus 87930
2508 DH Den Haag (NL)

Remarks:
This application was filed on 25 - 10 - 1999 as a divisional application to the application mentioned under INID code 62.

# (54) A method for inducing resistance of plants to mycotoxin-producing fungi

(57) A method for inducing resistance of plants to mycotoxin-producing microorganisms wherein at least one compound capable of inducing said resistance is produced in the plant of interest by modulating the expression of at least one gene of said plant, wherein said compound has a structure shown in formula (1):

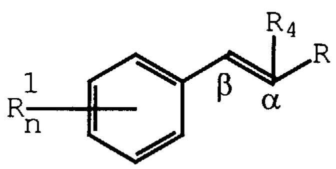

(1)

wherein

R represents $-CH_2OH$ or -CHO;
n is an integer from 0-3;
each $R^1$ independently represents OH or an organic substituent containing from 1-10 carbon atoms and from 0-5 heteroatoms, wherein the total number of carbon and heteroatoms in all $R^1$ substituents of said compound is no more than 15; and
$R_4$ represents hydrogen or an organic substituent containing from 1 to 10 carbon atoms,

wherein said compound is non-phytotoxic to said plant.

EP 1 000 543 A1

## Description

The invention relates to a method for inducing resistance of plants to mycotoxin-producing fungi, or for controlling the level of toxic metabolites in plants, and especially in consumable products by modulating the expression of genes of plants so that these plants will produce certain compounds. The present application is a divisional application of European patent application 95 944684.0 and the contents thereof are incorporated herein by reference.

Mold induced contamination and deterioration of agricultural products causes economic loss and poses health hazards. Fungi from the genera *Aspergillus, Alternaria, Fusarium, and Penicillium* can contaminate food crops and their products. Mycotoxins produced by these fungi include aflatoxins, fumonasins, fusaric acid, TA/AAL toxins, zearalenone, and trichothecene, 5-butylpicolinic acid and related phytotoxic pyridine derivatives. These mycotoxins are highly toxic to a variety of species including humans and can be found in commercially prepared food stuffs and animal feed. Serious health problems may arise when fowl, fish animals or humans ingest materials that have been contaminated with certain genera of fungi which produce mycotoxins.

Mycotoxins are secondary metabolites; these produced by various species of *Aspengillus* are the best known. As an example, *Aspergillus flavus* grows on a variety of plant materials and produces a low molecular weight mycotoxin, aflatoxin, that is poisonous to humans and many other animal species. The real and potential danger of these toxins was dramatically shown in 1960 by a large-scale trout poisoning in commercial fish hatcheries caused by fish food contaminated by fungi. The fumonisins also have been shown to affect the growth and health of domesticated animals of commercial value including horses, pigs, chickens and turkeys. Fumonisins also have been identified in food stuffs used to feed animals employed broadly in biomedical research and commercial testing. Research therefore may be compromised when experimental animals are reared on food variably contaminated with mycotoxins. Fumonisin represents a class of mycotoxins which are contaminants found in certain food crop products. Also, the same Fusarium fungi which colonize stored corn and corn products produce fusaric acid. The Fusarium mycotoxins are heat stable and survive extensive processing and are found in many corn products, including fructose corn sweetener, the major source of sweeteners in the food industry, and commercially produced fermentation products such as grain alcohol. Other mycotoxins which have been identified in corn materials and other vegetable material include, TA/AAL toxins (structure analogs of fumonisin), aflatoxins, zearalenone, trichothecene, 5-butylpicolinic acid and related phytotoxic pyridine derivatives.

Fusaric acid has been shown to affect neurotransmitters, therefore nerve function of both the central and peripheral nervous systems and heart function may be affected upon exposure to fusaric acid and fumonisin-contaminated materials and products. Thus, elimination of fusaric acid and other mycotoxins that have yet to be identified from consumable materials may play a significant part in well-being of animals and humans alike.

The presence of mycotoxins in food is attributable to the wide-spread distribution of fungi and their growth during storage and handling of contaminated food and crops. High levels of all types of mycotoxins have been found in a variety of edible commodities including beans, cereals, coconuts, peanuts, sweet potatoes and commercially prepared animal feeds. Mycotoxins also have been identified in milk and milk products. Outside of using chemical preservatives, pasteurization or tightly controlled and dry storage conditions, steps to prevent or reduce the level of mycotoxins present in these commercial products have proved ineffective.

Mycotoxin contamination of tobacco products may be linked to the pathology of diseases associated with tobacco consumption. Tobacco plants are often severely contaminated with fungi that produce mycotoxins, including the fumonisin and fusaric acid producing fungi. Fumonisins have been implicated in esophageal cancer in humans, the third most frequent cancer world-wide. Both fumonisin and fusaric acid producing organisms are present on tobacco and thus may play an important, and heretofore unrecognized role among consumers of both cigarette and smokeless tobacco, in oral, throat, and lung cancers, as well as other diseases associated with tobacco consumption. Tobacco also has been shown to be contaminated with other mycotoxins including the aflatoxins.

Chemically, mycotoxins are heat stable compounds of relatively low molecular weight. Thus, once the food or other consumables become contaminated with unacceptably high levels of mycotoxin, the product generally must be discarded. It therefore is of interest to develop methods that can be used to prevent contamination of consumables, and to identify the presence of mycotoxins in a product, to detoxify toxin-contaminated non-consumable materials to decrease the negative health affects associated with exposure to these materials. Moreover, it is highly desirable to control the level and toxic effects of microbial toxins that accumulate before, during and/or after consumable products enter the food chain or are otherwise consumed.

The presence of mycotoxins in stored leaves of chewing tobacco is described in Varma *et al., Mycopathologia (1991), 113:19-23*. The antibotulinal properties of various aromatic and aliphatic aldehyde are disclosed in Bowles and Miller, J. Food Protection (1993) 56:788-794. Other formulations which include cinnamic aldehyde have been reported to protect crops from attack by pathogenic microbes. See U.S. Patent Nos 4,978,686 and 5,149.715 and French patent application 2529755. Film-forming and/or antitranspirant coating polymers such as sodium bicarbonate and light paraffinic petroleum oils have been reported to control the level of fungal colonization. Horst *et al. (Plant*

*Disease,* March 1992, p.247), Elad *et al.* *(Phytoparasitica* (1989) 17:279-288) and Hagiladi *et al.* *(J. Environ. Hortic* (1986) 4:69-71). Protection of farm products from insects, microbes and bacteria to prevent physical harm to the farm products using an emulsion of cinnamic aldehyde is reported in Japanese patent application 814965. The formulation is reported to be rapid acting and to leave no residue.

In accordance with the present invention it has now been found that resistance to mycotoxin producing microorganisms can be induced by modulating the expression of at least one gene of said plant to produce at least one compound capable of inducing said resistance.

According to the present invention a method is provided for inducing resistance of plants to, or providing biocontrol of, mycotoxin-producing microorganisms wherein at least one compound capable of inducing said resistance, or controlling the growth of said microorganisms, is produced in the plant of interest by modulating the expression of one or more genes of said plant, wherein said compound has the formula (1):

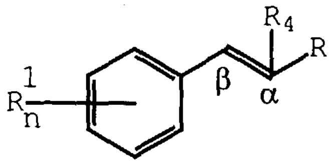

(1)

wherein

- R represents $-CH_2OH$ or -CHO;
- n is an integer from 0-3;
- each $R^1$ independently represents OH or an organic substituent containing from 1-10 carbon atoms and from 0-5 heteroatoms, wherein the total number of carbon and heteroatoms in all $R^1$ substituents of said compound is no more than 15; and
- $R_4$ represents hydrogen or an organic substituent containing from 1 to 10 carbon atoms,

wherein said compound is non-phytotoxic to said plant. Compounds of formula (1).

Preferred compounds are given in formula (2):

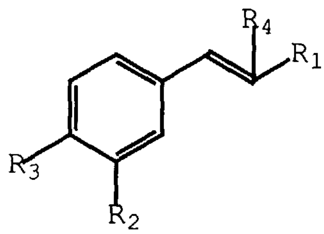

(2)

wherein

- $R_1$ represents -CHO;
- $R_2$ represents hydrogen, a methoxy group or an organic substituent containing from 1 to 10 carbon atoms;
- $R_3$ represents hydrogen, OH or an organic substituent containing from 1 to 10 carbon atoms; and
- $R_4$ represents hydrogen or an organic substituent containing from 1 to 10 carbon atoms.

Of particular interest are aromatic aldehydes. Examples of aromatic aldehydes of use in the present invention are cinnamic aldehyde, the structure of which is shown in formula (3):

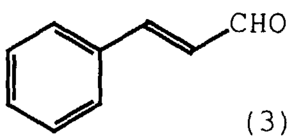

(3)

and coniferyl aldehyde, the structure of which is shown in formula (4):

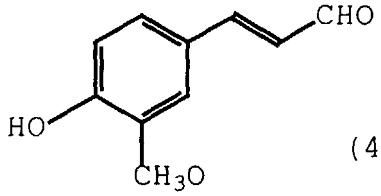

(4)

Other compounds of interest include compounds of formula (1) substituted at the alpha position with an alkyl, such as an hexyl group, or a branched alkyl group, such as an amyl group. Generally the group at the alpha position is from C-5 to C-10. Such compounds include α-hexyl cinnamic aldehyde (α-HCA) and α-amyl cinnamic aldehyde (α-ACA). The structure of α-HCA is shown in formula (5):

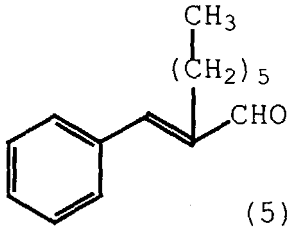

(5)

According to the invention, the expression of one or more genes encoding one or more enzymes or an enzyme pathway or cluster is modulated. In this way the level of the compound of interest in the plant, viz., the plant part, the plant cell or specific plant tissue, can be controlled. The expression may be associated with a particular stage of plant growth.

The present invention relates to methods for controlling the level of toxic metabolites present in e.g. a variety of consumable products that are either colonized or capable of being colonized by toxin-producing microorganisms. The method includes the production in the plant of a compound particularly an aromatic aldehyde, which limits the colonization of, kills or displaces one or more microorganisms which colonize the consumable material or precursor and which produce toxin(s). The invention finds use in controlling the level of toxic metabolites present in consumable products derived from plant materials, as well as decreasing contamination of the food chain by fungal toxins and toxic metabolites.

Methods are provided for modulating the level of one or more toxic metabolites associated with consumable products, particularly agricultural products that are capable of being colonized by one or more toxin-producing microorganisms. The methods involve killing or long term displacement of one or more toxin producing microorganisms, particularly using naturally occurring compounds such as the aromatic aldehydes cinnamic aldehyde, coniferyl aldehyde and alpha hexyl cinnamic aldehyde (HCA). The invention is particularly suited for reducing the level of mycotoxins and other toxic secondary metabolites associated with plant parts such as stems, leaves, roots, fruit, seeds, and/or flowers before, during and/or after the plant and/or plant part is harvested and/or processed for consumption. The present invention further provides assays for the detection and quantitation of microbial toxins present in a sample in order to determine if and the extent to which a sample is contaminated. The assays are used to test for toxin levels associated with whole plants and other materials under field or non-field conditions, during or after a plant or plant part is harvested and/or processed into a consumable product. Additionally, the assays may be used to identify toxic secondary metabolites produced by certain species of fungi, for example, species of *Fusarium,* that may be useful as herbicides, insecticides, fungicides, and or as pharmacological agents.

The invention offers several advantages over currently available detoxification techniques. One advantage is that contamination of consumable agricultural products can be prevented or significantly decreased to a level safe for consumption. By treating a plant in the field with a substance which kills or displaces mycotoxin-producing fungi, the levels of toxin contamination can be significantly reduced in the harvested material. The aromatic aldehydes in particular have positive organoleptic and olfactory properties which in some cases may improve the flavor and/or smell of treated products. The odor of HCA for example is described as floral or jasmine-like with some herbaceous character (Technical Data Sheet).

A number of the aromatic and aliphatic aldehydes which may find use in the subject invention, such as benzaldehyde, acetaldehyde, cinnamaldehyde, piperonal, and vanillin are generally regarded as safe (GRAS) synthetic flavoring agents (21 CFR §172.515). HCA was in public use before the 1950's and today is widely used in consumer preparations (soaps, detergents, creams, lotions, perfumes) (Monographs on fragrances raw materials. Food Cosmet. Toxicol. 12: suppl., 915, 1974). HCA was granted GRAS (generally recognized as safe) status by FEMA (Flavoring Extract Manufacturers' Association. Survey of flavoring ingredient usage levels. No. 2569. Fd. Technol., Champaign, 19: (part 2)155, 1965) in 1965 and is approved by the US FDA for use in food (21CFR121.1164). The Council of Europe (1970) (Council of Europe. Natural and Artificial Flavoring Substances. Partial Agreement in the Social and Public Health Field. Strasbourg, List A(1), Series 1, no. 129, p. 55, 1970) included HCA in the list of admissible artificial flavoring substances at a level of 1 ppm. Various of these compounds have been reported to have inhibitory activity against *C. botulinum spore germination.* Bowles and Miller, *G. Food Protection* (1993) 56: 788-794.

Surfactants which can be used as emulsifiers such as saponin (which also has GRAS status) also already are used as food additives. In addition, formulation residuality can be managed. This will be of great benefit when short term residuals are desired for integrated pest management programs with beneficial insects. In addition, the formulations work against pests which are resistant to other agents and are effective on multiple target organisms, including not only mycotoxin producing fungi but also insect targets. This reduces the need for application of multiple agents to the consumable material of interest. The effects of the formulation are long lasting. The long term control of pathogenic organisms results in a healthier plant and an improved yield of produce of the host plant as compared to untreated plants} the lower concentrations of antipathogenic agents decrease the likelihood of damage to the plant or its crop as well as decrease the likelihood of any adverse side effects to workers applying the pesticide, or to animals or fowl which ingest the tissues or parts of treated plants.

Phytotoxicity of the formulation also is decreased when the antioxidant is eliminated. Reentry time to the greenhouse also is not an issue. Typically the formulations are rapidly lethal to a target organism; this is a particularly valuable characteristic when coupled with no reentry time, (for example, no loss of cut flower inventories).

For materials which are used as food stuffs for humans, or for feed for food commodities consumed by humans, there is an additional advantage in that toxins are decreased or eliminated in the food chain. As an example, the meat from fish, fowl and animals which have fed on materials such as contaminated grain or contaminated dried grasses also is contaminated by toxins. Although an aromatic aldehyde, cinnamic aldehyde. has been reported to exhibit antifungal properties, it has not previously been used on plants in a formulation which is intended to provide long-term protection to a plant so that mycotoxin contamination is avoided a consumables produced from the plant. not only fresh fruit or fresh vegetables, tobacco, and food grains for fowl, animals, fish, and other elements of the human food chain. Thus, treatment of the materials on which the animals feed decreases or eliminates toxins in the food chain. Another advantage of the invention is that environmental exposure of animals and humans to, for example, water and airborne sources of mycotoxins resulting from the burning and/or disposing of contaminated silage materials of all types is reduced by detoxification of the agricultural materials either before or after harvest by treatment to decrease or eliminate the fungi which produce the toxins. The detoxification procedures thus allow for the safe disposal of materials such as plants, plant materials and foodstuffs derived therefrom.

In accordance with the invention, natural compounds can be used to kill or displace toxin-producing organisms from the plants or plant parts that they colonize, thereby limiting the amount of toxin that normally accumulates on the material. The compound is preferably biodegradable and is produced optionally together with a growth promotant and surfactant such as saponin, which can be derived from the *Yucca shidigera* plant. The susceptibility of particular fungi to the composition can be evaluated either *in vitro or in vivo.*

Of particular interest are various aldehydes, particularly aromatic aldehydes which can be used for direct killing of fungal pathogens and/or for the induction of systemic plant resistance to various fungal pathogens. The method includes the step of providing to one or more parts or tissues of a plant susceptible to attack by pathogens with an antipathogenic agent in an amount sufficient to control growth of target pathogenic organisms. The growth modulating product has a formula shown in (1) below.

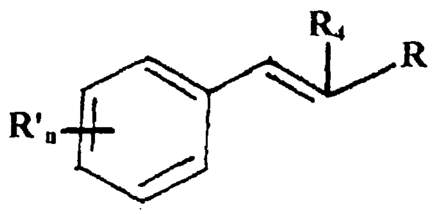

(1)

wherein R represents $-CH_2OH$ or -CHO: n is an integer from 0 to 3: each $R^1$ independently represents OH or an organic substituent containing from 1 to 10 carbon atoms and from 0 to 5 heteroatoms, wherein the total number of carbon and heteroatoms in all $R^1$ substituents of said compound is no more than 15: and $R^4$ represents hydrogen or an organic substituent containing from 1 to 10 carbon atoms. These compounds include natural compounds such as cinnamaldehyde, coniferyl aldehyde, and closely related compounds and provide a method to biocontrol pathogen infestations. By "biocontrol" is intended control of plant pathogens via direct antipathogenic activity and/or induced resistance of the host plant to pathogen infestation. By "colonizing" is intended association of a microorganism or insect with a plant part or tissue from which the pathogen derives nutrients, typically essential nutrients such as amino acids, particularly methionine. By "natural product' is intended an organic compound of natural origin that is unique to one organism, or common to a small number of closely related organisms, and includes secondary metabolites of fungi and chemicals produced by plants. By "provided with" is intended induction of synthesis in the plant of antifungal compounds, either compounds endogenous to the plant and/or compounds supplied by genetic manipulation. Genetic manipulation can be performed by traditional cross-breeding methods or by introducing transgenes into the plant or an ancestor of the plant using recombinant DNA technology. The natural products can be isolated from a natural source, be wholly or partially synthetic, or be produced by recombinant techniques either in the plant itself or in another organism

A preferred formulation is shown in formula (2) below.

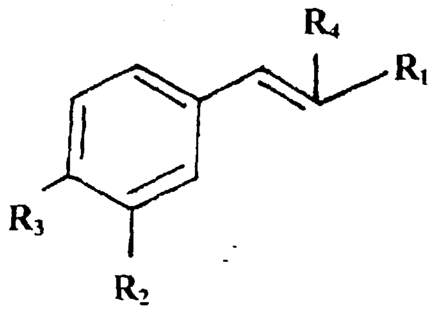

(2)

wherein $R_1$ represents-CHO. $R_3$ represents -H. -OH or an organic substituent containing from 1 to 10 carbon atoms. $R_2$. represents -H. a methoxy group or organic substituent containing from 1 to 10 carbon atoms; and $R_4$ represents hydrogen or an organic substituent containing from 1 to 10 carbon atoms. Of particular interest are aromatic aldehydes. Examples of aromatic aldehydes of use in the present invention are cinnamic aldehyde ((3) below).

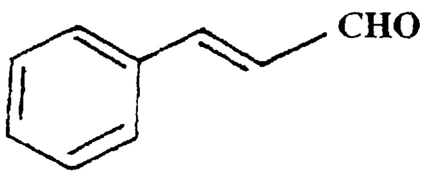

(3)

and coniferyl aldehyde ((4) below).

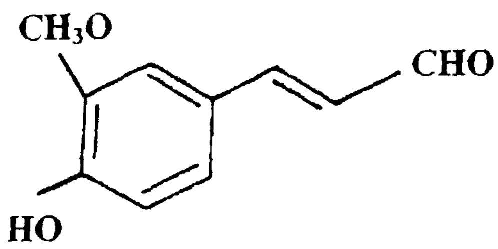

(4)

Other compounds of interest include analogs of the compound of formula (1) such as compounds substituted at the alpha position with an alkyl, such as a hexyl group, or a branched alkyl group such as an amyl group. Generally the group at the alpha position is from C-5 to C-10. Such compounds include alpha hexyl cinnamaldehyde and alpha amyl cinnamaldehyde. The chemical structure of alpha-hexylcinnamic aldehyde is shown in (5) (below).

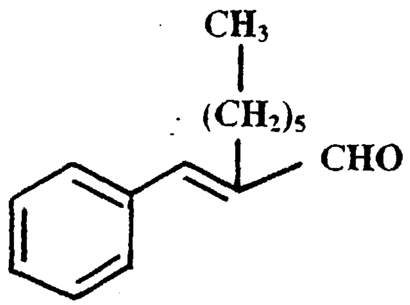

(5)

The Chemical Abstracts Service (CAS) name is 2-(phenylmethylene) octanal and the CAS Registry Number is [101-86-0]. The compound is also described by the chemical name of 2-hexyl-3-phenyl-2-propenal. The compounds's formula is $C_{15}H_{20}O$ and molecular weight is 216.3. HCA can be obtained from Firmenich; their product is composed principally of the (E)-cis isomer (93.8% maximum), and the (Z)-trans isomer (6% maximum). Among minor components is the self aldol condensation product of octanal (1-1.5 % (Personal Communication. June Burkhardt, Firmenich, Plainsboro, New Jersey).

The compounds may be used either alone or in combination with other active or inactive substances and may be applied by spraying, pouring, dipping, in the form of concentrated liquids, solutions, suspensions, powders and the like, containing such concentration of the active compound as is more suited for a particular purpose at hand. They also can be applied, for example, in the form of dilute solution, in a suitable solvent directly to the rhizosphere either as part of an irrigation schedule or as a separate application.

For use as a foliar spray, although the aldehyde can be formulated alone, it can be rendered substantive by including a sufficient amount of an emulsifier such as Tween 80, or a compound such as saponin which has surfactant properties but does not significantly impact the antifungal properties or the formulation. Generally, detergents in the formulation do not detract from the antifungal properties of the aromatic aldehydes but do increase the substantive properties of the formulation. *See* for example. U.S. Patent No. 4,477.361.

Other detergents which can be used include anionic detergents such as those described in U.S. Patent No. 4,978,686. Additional components such as an aqueous preparation of a salt of a polyprotic acid such as sodium bicarbonate, sodium sulfate, sodium phosphate or sodium biphosphate can be included in the formulation, to increase the antifungal properties of the formulation. The resulting emulsion is diluted to an appropriate concentration for use.

In a preferred embodiment, the formulation includes alpha hexyl cinnamic aldehyde, cinnamic aldehyde and/or coniferyl aldehyde in a formulation containing Tween 80 or saponin as an emulsifier and optionally sodium bicarbonate. The preferred formulation is an emulsion which contains alpha-hexyl cinnamic aldehyde, cinnamic aldehyde and/or coniferyl aldehyde, 0.5% to 10% by weight and may include the salt of an aprotic acid, 8% to 12% by weight and the balance water. Formulations with 6-12% of an aprotic acid are preferred. Generally, the total amount of aldehyde(s) present in the formulation is 5% or less. The formulations are effective without the use of antioxidants other than the inherent antioxidant properties of particular aldehydes, for example, coniferyl aldehyde.

Stability of the formulation can be evaluated by a variety of methods, including accelerated tests in which a formulation of interest is exposed to elevated temperatures over a set time. Samples of the formulations are taken at regular intervals and analyzed chemically by methods known to those skilled in the art to determine the rate and nature of degradation. For example, HCA can be analyzed by Gas Liquid Chromatography (GLC), using a 30 meter non-polar polydimethylsiloxane capillary column (e.g. HP-1, Hewlett-Packard, or SPB-1, Supelco) and a flame-ionization detector. Using helium as a carrier gas (8 ml/min.) and a column temperature of approximately 240°C, the (E)-cis isomer (major component) has a retention time of approximately 6.0 minutes and the (Z)-trans isomer (minor component) has a retention time of approximately 6.3 minutes.

The most effective antifungal amount for compositions including compounds or formula (3) and/or formulas (4) or (5) as well as the amount of other compounds of formula (1) which may find use can be determined using protocols known to those skilled in the art. As an example, for each particular application the mean disease resistance can be calculated; generally for effective pathogen control the mean percentage of disease control (MPDC is greater than 60%, preferably at least about 70%. These protocols also can be used to optimize each formulation for specific pathogens using any of the compounds encompassed by formula (1). MPDC is defined by the formula:

$$MPDC = \left(\frac{MDIC\text{-}MDIT}{MDIC}\right) \times 100$$

and

MDIC = Mean % of disease incidence in untreated controls

MDIT = Mean % of disease incidence in the treatment

The formulations also need to be evaluated for phytotoxicity; it therefore is important that at least one evaluation of the toxicity of the formulations be on living plants of the host variety. Phytotoxicity can be rated as follows in order of increasing severity of toxicity: 0-plants without any symptoms; 1-very slightly browning of hypocotyl (no other symptoms); 2-some wilting of plant, dying of lower leaves, some browning of vascular system; 3-wilting of entire plant, leaves dying, hypocotyl with external and internal symptoms; 4-necrosis of stem. plant dying. The phytotoxicity generally should be 2 or less, preferably 1 or less.

In some instances, the efficacy of the formulation can be increased by adding one or more other components to the formulation where it is desirable to alter particular aspects of the formulation. As an example, it may be desirable for certain applications to decrease the phytotoxicity effect when used pre-harvest or to increase the antipathogenic effect of the formulation or both. It is preferable that the other components minimize phytotoxicity while increasing the antipathogenic effect of the formulation. Of particular interest is the use of a component(s) to increase the mean disease resistance of a formulation against toxin producing microorganisms that are either colonized or capable of colonizing consumable product. The concentration of one or more of the other formulation ingredients can be modified to optimize the antipathogenic and reduce the phytotoxic effect of the formulation. Of particular interest is the addition of a component to the formulation to allow for an overall reduction in the concentration of one or more other ingredients in a given formulation, particularly components according to formula (1), while maintaining overall efficacy of the formulation. Combination of such a component with other ingredients may be accomplished in one or more steps at any suitable stage of mixing and/or application.

Preferred additional components include saponins. Saponins are a class of compounds, each consisting of a sapogenin portion and a sugar moiety. The sapogenin may be a steroid or a triterpene and the sugar moiety may be glucose, galactose, a pentose, or a methylpentose. S. Budavari, ed., *The Merck Index,* 11th ed., Merck & Co., Inc., Rahway, N.J., 1990, p. 1328. The saponins for use in the present invention can be produced and/or isolated from various plant pans including fruit, leaf, seed and/or root, using means known in the art, from a variety of sources including the various plants known to produce them, ranging from yucca, quillaja, agave, tobacco, licorice, soybean, ginseng and asparagus to aloe woods. Saponins for use in with the present invention are preferably non-toxic to humans and higher animals. Most preferably the saponin for use in the present invention is non-toxic food grade, the source being from yucca plants. Even more preferred are the saponins from *Yucca schidigera or Y. valida* and their equivalents. The more preferred saponins for use in the present invention are derived from yucca plants, with the most preferred being *Yucca schidigera or Y. valida.*

A variety of structurally related saponins are known, the most variable structural feature being the glycosylation pattern. Saponins also may contain additional modifications, such as the sarasaponins which are saponins with a steroid attached, and saponin structure can be modified by any number of enzymatic, chemical and/or mechanical means known in the art. Saponins from *Yucca schidigera* contain steroidal saponins with the major sapogenins being sarsapogenin and tigogenin. The sarsaponin yields on hydrolysis, sarsasapogenin (sarsasapogenin 5-beta, 20-betaF, 22-deltaF, 25-betaF; also known as spirostan-3-beta-01 and parigenin), glucose and galactose. The sarasapogenim has a molecular formula of $C_{27}H_{44}O_3$. Nobel, Park S., *Agaves,* Oxford Univ. Press, New York, 1994.

Accordingly, derivatives of these compounds which produce a formulation having the desired antipathogenic and/or phytotoxic effect are considered equivalents of the invention. Depending on its structure, a given saponin can have a particular pesticidal property and lend use with the present formulations. Generally an effective amount of saponin is of the range of about 0.01 to 3% and most preferably about 0.25% v/v aqueous solution of 10° brix saponin extract. The brix degrees equals the percent by weight of sugar in the solution. Hawley, ed., The Condensed Chemical Dictionary, 10th ed., Van Nostrand Reinhold, New York, 1981, p. 149.

Each formulation is evaluated for its effect on specific toxin producing microorganisms and/or consumable product pre- or post-harvest such as a plant host or plant product using any of the compounds of formula (1) as well as other components with the combination and effective amount of each component adapted for a particular application to minimize toxicity while maintaining or increasing the antipathogenic effect of the formulation. The effective amount of each component may be determined by systematically varying the amount of that component in a test formulation, treating a crop of interest with the test formulation, and monitoring the mycotoxin levels in the plant host or plant product

pre- and post-harvest and after a period of storage. An effective amount of a test component may be identified as the amount that controls the residual mycotoxin present in the plant product at a level acceptable for the particular crop of interest.

In addition to the specific compounds of the formulas (1), (2), (3), (4) and (5) and optionally saponin as set forth above, derivatives of any of these compounds that produce a compound of the formula identified above upon action of a biological system on the derivative are considered to be equivalent to compounds of the invention. Thus application of precursor compounds to plant parts or tissues or harvested materials would be equivalent to the practice of the present invention. Biological conversion of precursor compounds into aromatic aldehydes is described in, for example, U.S. Patent Application No. 5,149,715 and references cited therein. *See* also Casey and Dobb *Enzyme Microb. Techol.* (1992) 14: 739-747. Examples of precursor compounds include those in pathways relating to acquired and/or systemic resistance to plant resistance to plant pathogens such as those in the amino acid ammonia lyase pathways, and include phenylalanine ( to produce cinnamic acid). Other precursor compounds of interest include those in the biological pathways for the of lignins and coumarins, for example tyrosine to produce *p*-coumaric acid. Accordingly, precursors and derivatives of these compounds which produce a formulation having the desired antipathogenic and toxin reducing effect are considered equivalents of the invention.

Depending upon the target organism, the aldehyde to be used can be coupled to a solid support, optionally through a linker such as a binding domain derived from a polysaccharidase, where the solid support is a polysaccharide such as cellulose, particularly microcrystalline cellulose. The preparation of cellulose binding domains is described in U.S. Patent Nos. 5,340,731; 5,202,247 and 5,166,317. The aldehydes can be coupled to the binding domains, with or without a cleavable bond, using methods well known to those skilled in the art. Binding domains from scaffold proteins also can be used. *See* Shoseye*n et al.* (PCT application PCT/0594/04132).

Other compounds can be used alone or in combination with the compositions to prevent the accumulation of toxins, and/or kill or displace one or more toxin producing microorganism, for example $H_2O_2$, which is known to kill particular fungi such as white rot fungus. Additionally, for use pre-harvest, compounds which induce non-systemic or systemic plant resistance to various fungi can be used to control colonization of certain fungi under field conditions. When a plant material is heavily contaminated with microbial toxin, for example, disposable or composted plant materials, the material additionally can be treated with an effective amount of an alkaline-peroxide or similar chemical which destroys or neutralizes the toxin present in the material. A mechanical process can be used separately or in combination with the chemical to promote the detoxification process. Examples of mechanical detoxification methods include any means that can be used to separate the toxin from the material or other means which destroys the toxin such as exposure to high heat and pressure.

If it is anticipated that a material may become contaminated with toxin, or if it is determined that the level of microbial toxin in a contaminated material makes the material unsuitable for safe consumption, the level of toxin can be controlled or reduced by treating the material so that the level of toxin is reduced to a level safe for consumption. More particularly, to reduce or counteract toxicity of microbial toxin-contaminated materials, an effective amount of one or more substances may be added to a product so as to counteract or preempt the toxic activity of the toxin. Effective substances include those containing thiols or sulfhydryl group amino acids, and sulfur amino acids for example cysteine, methionine and their derivatives. A preferred source of such amino acids can be chicken feather, hair, hides, hooves and other keratin animal products that have been treated so as to release the amino acids associated with them. Such treatment includes solubilization of keratin substrates in an alkaline-hydrogen peroxide (*e.g.,* NaOH + $H_2O_2$) environment. In a preferred embodiment, the alkaline/peroxide concentration is about 0.5-2.5%, but may be higher or lower as appropriate. Most preferably, the concentration of alkaline/peroxide is around 1-2%. Addition of sulfur amino acids to foods and feed counteracts the presence of mycotoxins, thereby reducing the potential health hazard associated with the consumption of these products.

The method of the present invention is carried out by introducing into a target pathogenic organism a sufficient amount of an antipathogenic agent to impair growth and/or viability of the target pathogenic organism. A formulation containing the antipathogenic agent is introduced to a plant tissue or part either pre- or post-harvest. For example, the formulation is sprayed on as a wet or dry formulation to the surface and/or underside of the leaves or other plant tissue or part of a plant infected with a plant pathogen, or of a plant susceptible to infestation with a plant pathogen, preferably to the point of run off when a wet formulation is used. The plants can be sprayed prior to or after infestation, preferably prior to infestation. However, in order to minimize damage to the host plant, where feasible, it is preferable to treat older plants, as young green leaves tend to be more susceptible to phytotoxicity. A plant growth promotant, such as saponin, is optionally used pre-harvest either in the antipathogenic formulation or as a separate formulation. Alternately, the formulation can be applied wet or dry to the rhizosphere where it can contact the roots and associated pathogenic organisms which colonize the roots. In some instances, time-release formulations may find use, particularly for applications to the rhizosphere, or to post-harvest materials.

The method of introducing the active ingredient(s) of the formulation into the target organism can be by direct ingestion by the pathogenic organism from a treated plant surface, or by feeding of a pathogenic organism on a

nutrient-providing surface of a host entity, which is colonized by the target pathogenic organism, which host either contains or has on its surface the antipathogenic agent.

The presence of the anti-pathogenic agent on a nutrient-providing surface of a host plant can be a result of direct contact of the antipathogenic agent with the plant part or it can be by elaboration from the host plant as a result of induction of systemic resistance as a secondary effect to prior treatment of the plant with the antipathogenic agent, or as a result of genetic modification of the host plant.

The aromatic and aliphatic aldehydes of the subject invention may be prepared by various synthetic methods known to those skilled in the art. For example, *see,* J. March, ed., Appendix B, *Advanced Organic Chemistry: Reactions, Mechanisms, and Structure,* 2nd Ed., McGraw-Hill, New York, 1977. Cinnamaldehyde may be prepared synthetically, for example, by oxidation of cinnamyl alcohol (Traynelis *et al., J. Am. Chem. Soc.* (1964) 86:298) or by condensation of styrene with formylmethylaniline (Brit. patent 504,125). The subject aldehydes may also be obtained by isolation from natural sources. For example, cinnamaldehyde may be isolated from woodrotting fungus, *Stereum subpileatum.* Birkinshaw *et al., Biochem. J.* (1957) 66:188.

HCA can be synthesized as described, for example, in USPN 5,055,621. On a laboratory scale, HCA can be synthesized by reaction of benzaldehyde with octanal under a nitrogen atmosphere (aldol condensation) (Personal Communication, Eric Walborsky, Firmenich Chemical Manufacturing Center, Port Newark, New Jersey). The reaction is conducted in a stirred flask charged with methanol, 309 ppm diphenylamine, potassium hydroxide and benzaldehyde. Following the slow addition of octanal, the reaction mixture is brought to a pH of 7.5-9.5 with acetic acid. Following evaporation of methanol and wash of the reaction mixture with water, the organic phase is transferred to a distillation unit. Approximately 20-24% of the pot charge is removed as benzaldehyde and "lights", with the remaining distillate constituting alpha-hexylcinnamic aldehyde "heart cut." The "heart cut" is subjected to an additional fractionation, in which 1-5% (by weight) of the material may be removed in "light" fractions, depending upon odor evaluation. The commercial process differs from the laboratory scale procedure in that diphenylamine has been replaced by a proprietary catalyst[2]. The final product is a light yellow oil having a specific gravity of 0.955-0.965 at 20°C, a refractive index of 1.548-1.562 at 20°C, a boiling point of 305°C at 1 atmosphere, and a melting point of 26°C. The commercial product is stabilized with the addition of 0.04% 2, 6-di-tert-butyl-p-cresol (butylated hydroxytoluene or BHT), which serves as an anti-oxidant (Technical Data Sheet, Hexylcinnamic aldehyde 907600, Revision 853, Firmenich Inc., Plainsboro, New Jersey). HCA can also be isolated from rice where it has been reported to occur naturally. (Givaudan-Roure Index, Givaudan-Roure Corporation, Clifton, New Jersey, 1994, p. 89).

HCA is a low to moderately volatile compound, having a vapor pressure of 70 x $10^{-5}$ mm Hg at 25°C. Its parent compound, cinnamic aldehyde, has a vapor pressure approximately 40 times higher (2970 x $10^{-5}$ mm Hg at 25°C). For comparison purposes, the insect repellant N,N-diethyl-m-toluamine has a slightly higher vapor pressure (167 x $10^{-5}$ mm Hg at 25°C) (Reifenrath, W.G. (1995) *Volatile Substances, Cosmetics and Toiletries,* 110: 85-93).

One or more components of the present formulations can be produced in the plant of interest by modulating the expression of one or more genes encoding one or more enzymes or an enzyme pathway or cluster required to control the level of the compound of interest in the plant, plant part, plant cell, specific plant tissue and/or associated with a particular stage of plant growth. The enzyme can be in a biosynthetic pathway or a degradation pathway and the regulation will be up or down, respectively, to modulate expression of either an endogenous plant gene or a transgene supplied exogenously to the plant. An indigenous plant gene is one which is native to the genome of the host plant. An endogenous plant gene is one that is present in the wild-type genome of the plant host of interest. It may be an indigenous gene or a gene that is present as a result of infection of the plant (for example, a viral gene) or otherwise naturally incorporated into the plant genome. The host plant also can be modified by recombinant means or by traditional plant breeding methods to introduce one or more genes exogenous to the host plant which encode or which control the level of the compound of interest and as such are in the synthetic pathway for one or more compounds of formula (1), (2), (3), (4) or (5). By modulation of gene expression is intended control of production of a gene product of interest at the level of transcription, translation and/or post translation. The level of the compound of interest is controlled by modulating the expression of one or more endogenous genes or transgenes encoding one or more enzymes required to synthesize the compound of interest.

Methods for modulating gene expression in plants are known in the art. Variation in growth conditions or exogenous application of compounds to a plant can affect gene expression. For example, the formulations of the present invention can be used to induce systemic plant resistance through modulation of endogenous gene expression. At the molecular level, gene expression depends substantially on the transcription, translation and termination control regions which regulate expression of a structural gene coding region. By exploiting the plant signals which regulate these control regions or by the direct recombinant manipulation of the control regions, expression of a gene encoding an enzyme required to control the level of cinnamic aldehyde, for example, can be modulated. For use in a transgene supplied exogenously to a plant host, the transgene will include control regions that are selected and designed to achieve the desired level and timing of gene expression. As appropriate, the control regions may be homologous (native) or non-homologous (non-native) to the gene of interest. By "homologous" it is meant that the control region(s)

is from or substantially similar to a control region normally associated with the gene of interest. By "non-homologous" it is meant that the control region(s) originates from a different nucleotide source or sequence or is substantially different from the control region(s) normally associated with the gene of interest. For example, if the enzyme coding sequence is non-homologous in source as compared to the control regions, in order to have expression of the gene in a plant cell of interest, transcriptional and translational initiation regulatory regions or promoters functional in these plant cells must be provided operably linked to the coding sequence. Transcription and translation initiation signals functional in plant cells include those from genes which are present in the plant host or other plant species, and direct constitutive or selective expression in a plant host.

Of particular interest are the gene control regions that selectively regulate structural gene expression in a plant, plant pan, plant cell, specific plant tissue and/or associated with a particular stage of plant growth. Preferred are those control regions that are known in the art, and in particular, transcriptional control regions or promoters that can be used to modulate the expression of a gene encoding an enzyme required to control the level of a component of formula (1), (2), (3), (4), (5) and/or saponin in a plant, plant pan, plant cell, or specific plant tissue and/or are associated with a particular stage of plant growth. For example, promoters providing for differential expression patterns in fruit are described in USPN 4,943,674 and USPN 5,175,095; seed in USPN 5,315,001; rapidly developing tissues and tender shoots in USPN 5,177,011.

A preferred method for producing a desired component of the present formulations in a plant host is through recombinant DNA means, particularly by modifying the level of at least one compound of interest of the formula (1), (2), (3), (4), (5) and/or saponin in plant tissues of interest through construction of transgenic plants using recombinant techniques known in the art. The methods involve transforming a plant cell of interest with an expression cassette functional in a plant cell comprising as operably linked components in the 5' to 3' direction of transcription, a transcriptional and translational initiation regulatory region, joined in reading frame 5' to a DNA sequence encoding one or more enzymes capable of modulating the production and/or required to produce the compound of interest, and translational and transcriptional termination regions. Expression of an enzyme required to produce the compound of interest provides for an increase in production of the compound as a result of altered concentrations of the enzymes involved in the compounds' biosynthesis. Of particular interest is the selective control of saponin, cinnamic and/or coniferyl aldehyde production in plant tissues such as leaves, roots, fruits and seeds.

For cinnamic aldehyde biosynthesis in a tissue of interest, plant cells are transformed with an expression cassette comprising DNA encoding a structural gene for one or more enzymes required to synthesize cinnamic aldehyde and capable of increasing the amount of cinnamic aldehyde in the tissue of interest. Similarly, for selective control of saponin biosynthesis in a tissue of interest, plant cells are transformed with an expression cassette comprising DNA encoding a structural gene for one or more enzymes required to synthesize saponin and capable of increasing the amount of these compounds in the tissue of interest. Of particular interest are those genes encoding one or more enzymes capable of metabolizing a precursor compound required for the biosynthesis of the saponin. cinnamic and/or coniferyl aldehyde compound of interest from substrates normally found in a plant cell. More particularly is the transgenic expression of at least one compound of the formula (1), (2), (3), (4), (5) and a saponin.

DNA constructs for expressing a gene of interest are prepared which provide for integration of the expression cassette into the genome of a plant host. Integration can be accomplished using transformation systems known in the art such as *Agrobacterium.* electroporation or high-velocity microparticle-mediated transformation. Depending upon the application. saponin or one of the other compounds of interest can be preferentially expressed in a tissue of interest and/or a particular organelle. Tissue specificity is accomplished by the use of transcriptional regulatory regions having the desired expression profile. Translocation of the enzyme to a particular organelle is accomplished by the use of an appropriate translocation peptide. Methods for tissue and organelle specific expression of DNA constructs have been described are known in the art.

To verify regulation and expression of the gene of interest, various techniques exist for determining whether the desired DNA sequences present in the plant cell are integrated into the genome and are being transcribed. Techniques such as the Northern blot can be employed for detecting messenger RNA which codes for the desired enzyme. Expression can further be detected by assaying for enzyme activity or immunoassay for the protein product. Most preferably the level of the compound of interest present in a plant host is measured using methods known in the art. A desired phenotype, for example, is increased saponin and/or aromatic aldehyde content in a plant tissue of interest as measured by expression of the gene of interest and/or the level of saponin present in the plant host as compared to a control plant.

For introduction of one or more compounds of the present formulations to the target organism, a plant host expressing a gene encoding an enzyme required to control the level of the compound of interest results in the exposure of a target organism to at least one component of the antipathogenic formulation. In another embodiment, selective expression of the gene of interest induces systemic plant host resistance to pathogen attack or colonization. At least one component of the antipathogenic formulation can be expressed by the plant host and optionally other components of the antipathogenic formulation are exogenously applied to the plant host so that the combination elicits the desired

antipathogenic effect when either directly or indirectly introduced to the target organism.

Transgenic plants having an increased ability to accumulate aromatic aldehydes such as cinnamaldehyde and coniferyl aldehyde to provide self-protection against plant or be used as a natural source of aromatic aldehydes for extraction and subsequent use as a chemical pesticide can be prepared as follows.

Accumulation of aromatic aldehydes can be achieved by downregulating the expression of specific plant genes that encode enzymes which either cause further metabolism of the desired aldehydes or divert metabolic intermediates away from the desired aldehydes. In the case of cinnamaldehyde, for example, this involves downregulating the expression of cinnamate 4-hydroxylase (CA4H) and cinnamic alcohol dehydrogenase (CAD). CA4H ordinarily diverts some cinnamic acid away from cinnamaldehyde to produce *p*-coumaric acid. itself a metabolic intermediate. Reducing CA4H activity alone is not sufficient to cause accumulation of cinnamaldehyde because CAD can rapidly convert cinnamaldehyde to cinnamyl alcohol, which then becomes incorporated into lignin or accumulates as glycosides. Simultaneously reducing both CA4H and CAD activities results in increased metabolic flux from cinnamic acid into cinnamaldehyde and decreased conversion of cinnamaldehyde into cinnamyl alcohol. Some cinnamaldehyde becomes incorporated into lignin but cinnamaldehyde (either free or as glycosides) also accumulates to above-normal levels, particularly at times when the biosynthesis of cinnamic acid is elevated. This occurs when the level of phenylalanine ammonia lyase (PAL: the first and rate-limiting step in general phenylpropanoid metabolism. Hahlbrock and Scheel, (1989) *Annu. Rev. Plant Physiol. Plant Mol. Biol*. 40:347-369) activity is high. a situation that naturally occurs in plants in response to a wide range of stimuli including invasion by fungal pathogens and mechanical damage associated with wounding and insect feeding.
Inhibiting CAD activity in transgenic plants has been proposed as a method of reducing lignin synthesis in plants and thereby improving the digestibility of fodder crops (WO 93/05159). These experiments suggested that lignin biosynthesis had been altered qualitatively, but not necessarily quantitatively, but did not demonstrate or appreciate the desirability of accumulating cinnamaldehyde as a method of increasing protection against pathogens.

A number of plant CA4H and CAD genes have been cloned and their sequences are available from GenBank. Portions of these genes that include nucleotide sequences that are conserved between different plant species can be used directly in a plant expression vector (antisense or sense orientation) to suppress the expression of the corresponding endogenous genes (*e.g.,* Pear *et al., Antisense Res. and Develop.* (1993) 3:181-190, Napoli *et al., The Plant Cell* (1990) 2:279-289. More preferably, these conserved gene sequences are used to isolate CA4H and CAD cDNA clones from a cDNA library of the plant species that is to be modified. The resulting cDNA clones, or portions thereof, are then introduced into a plant expression vector (antisense or sense) and used to transform the plant(s) of interest. DNA constructs according to the invention preferably comprise a sequence of at least 50 bases which is homologous to the endogenous CA4H or CAD genes.

A recombinant DNA molecule can be produced by operatively linking a vector to a useful DNA segment to form a plasmid that can be used for plant transformation. A vector capable of directing the expression of RNA from a cloned portion of a gene is referred to herein as an "expression vector." Such expression vectors contain expression control elements including a promoter. Typical vectors useful for expression of genes in higher plants are well known in the art and include vectors derived from the Ti plasmid of *Agrobacterium tumefaciens* described by Rogers *et al., Methods in Enzymology* (1987) 153:253-277. A common promoter that is used to provide strong constitutive expression of an introduced gene is the cauliflower mosaic virus (CaMV) 35S promoter (available from Pharmacia, Piscataway, NJ). Either constitutive promoters (such as CaMV 35S) or inducible or developmentally regulated promoters (such as the promoter from a PAL gene or the endogenous CA4H or CAD genes) can be used. Use of a constitutive promoter will tend to affect functions in all parts of the plant, while use of an inducible or developmentally regulated promoter has the advantage that the antisense or sense RNA is only produced in the tissue and under the conditions it is required. The use of developmentally regulated promoters is preferred in the use of this invention because the down-regulation of phenylpropanoid biosynthesis is known to be capable of producing undesirable side-effects on the development of transgenic plants containing a heterologous PAL gene (Elkind *et al.,* (1990) *Proc. Nat. Acad. Sci.* 87:9057-9061.

A number of different transformation methods are available for the routine transformation of a wide range of plant species. One method that is particularly efficient for the transfer of DNA into dicotyledonous plants involves die use of *Agrobacterium.* In this method the gene of interest is inserted between the borders of the T-DNA region that have been spliced into a small recombinant plasmid with a selectable marker gene (for example encoding neomycin phosphotransferase II or phosphinothricin acetyltransferase). The recombinant plasmid is then introduced into an *Agrobacterium* host by transformation or triparental mating. The *Agrobacterium* strain carrying the gene(s) of interest is then used to transform plant tissue by co-culturing the bacteria with an appropriate plant tissue (*e.g.,* leaf disc). Transformed cells are selected in tissue culture using the appropriate selection agent and plants are then regenerated (*see* Horsch et al., (1985) Science 227:1229-1231. Other methods that have been used in the transformation of plant cells, and in particular the more recalcitrant crop plants, include biolistics and electroporation (for detailed protocols. *see Sanford et al.,* (1993) *Methods in Enzymology* 217:483-509: and Potter. (1993) *Methods in Enzymology* 217:461-478.

Once transgenic plants have been produced, conventional enzyme assays for CA4H and CAD are used to

determine the level of suppression of enzyme activity achieved in different transformants. It is likely that only a small fraction of the transformants produced will have a sufficiently low residual enzyme activity to cause the accumulation of aromatic aldehydes without also producing some undesirable side effects on plant development. For this reason, a preferred method of producing the desired transformants with both CA4H and CAD suppressed is to introduce the two genes separately into different transformants and then combine them by standard sexual crosses. This permits a larger number of combinations of level of gene suppression to be evaluated at the same time.

An alternative to overproducing aromatic aldehydes in transgenic plants is to use the plant genes to confer on a microbial host the capability of synthesizing specific aromatic aldehydes and/or saponins. The resulting microbes may be used either to produce the aromatic aldehydes in a fermentation system or as a natural delivery system of the aromatic aldehydes in viable or non-viable microbial preparations. Yeasts, especially *Sachoromyces cerevisiae,* are preferred organisms for this purpose because they have already been engineered for high-level expression of PAL (Faulkener *et al.,* (1994) *Gene* 143:13020) and a plant cinnamate 4-hydroxylase has been shown to function in yeast (Urban *et al.* (1994) *Eur. J. Biochem.* 222:843-850.

The expression of PAL introduces the capability to produce cinnamic acid from phenylalanine. Two additional enzymic steps are required to produce cinnamaldehyde from phenylalanine. In plants, these steps are catalyzed by the enzymes cinnamate:CoA ligase (CL) and cinnamoylCoA reductase (CCoAR) but as 4-coumarateCoA ligase (4CL) can also use cinnamic acid as substance (Knobloch, and Hahlbrock (1977) *Arch. Biochem. Biophys.* 184:237-248), 4CL can be used instead of CL. More than 20 cloned PAL genes and more han 6 4CL genes have been described in sufficient detail (GenBank) to facilitate their use in practicing the current invention. A gene for a CCoAR is obtained by applying standard gene cloning techniques to isolate a cDNA clone using as a probe sequence derived from the mino acid sequence of the N-terminus, or peptide fragments, of the purified protein. CCoAR has been purified and partially characterized from soybean cultures (Wengenmayer *et al,* (1976) *Eur. J. Biochem.,* 65:529-536; Luderitz and Grisebach (1981) *Eur. J. Biochem.* 119:115-124), spruce cambial sap (Luderitz and Grisebach. *supra*), poplar xylem (Sarni *et al.* (1984) *Eur. J. Biochem.* 139:259-265) and differentiating xylem of *Eucalyptus gunnii* ( Goffner *et al,* (1994) *Plant Physiol,* 106:625-632). The preferred method of purification is hat of Goffner *et al, (supra)* because it results in a single protein band on SDS-polyacrylamide gels that an be used for protein sequencing.

The cloned genes are introduced into standard expression vectors and used to transform a microbial host, preferably yeast, by standard transformation techniques such as electroporation (Becker and Guarante (1991*) Methods in Enzymol.* 194:182-187). Standard enzyme assays are used to confirm the functional expression of the engineered genes and assays for aromatic aldehydes are used to select strains with maximal production. Because aromatic aldehydes have antimicrobial properties it is preferred to use expression vectors that will cause expression of the introduced genes only late in the growth cycle or in response to a chemical inducer. It may also be desirable to grow the engineered microbial host in an immobilized whole cell reactor (*e.g.,* Evans *et al.* (1987) *Biotechnology and Bioengineering* 30:1067-1072) to prevent the aldehydes from accumulating in the culture medium.

Material in which the level of mycotoxin can be controlled can either be consumable or non-consumable and is preferably a plant or of plant origin, although any other material contaminated with fungi which produce microbial toxins or is capable of being colonized by or supporting the growth of toxin producing microorganisms can be treated. Of particular interest are crops intended for consumption by fowl, fish and animals, including humans, directly or indirectly. By "directly or indirectly" is intended that the crops could be ingested, for example, by humans (direct consumption), or that it is the nonhuman animal or fowl or fish which ingests the crop and is in turn ingested by humans (indirect consumption). Crops intended for consumption include tobacco, animal and fowl fodder, crops intended for processing into alcohol or food products such as corn syrup, and the like. Plants and plant materials colonized by toxin producing fungi include, for example, barley and other grasses, rice, corn, wheat, oats, hops, cassava, beans, potato, peanuts, sweet potato, tomato, sugar cane, coconut, citrus, grapes, sorghum, melons, cucumber, lettuce, spinach, artichoke, onion, tomato, strawberry and tobacco.

The level of mycotoxin also can be controlled in products derived from plant materials, such as processed juices, corn products such as high fructose corn syrup, oil, meal, starch, alcohol and products containing these and other corn derived ingredients and tobacco products such as cigars, cigarettes, and smokeless tobacco by inhibiting or preventing growth of toxin-producing fungi in the materials from which these items are produced either pre or post harvest. Similarly, microbial toxin levels also can be controlled in forage grasses such as fescue, bent grass, alfalfa, clover, and turf grasses and in commercially prepared animal feeds including those for cattle, sheep, pigs, and horses; fowl such as turkeys and chickens; fish such as trout, catfish and salmon; domestic pet foods including dog and cat foods; and laboratory animal foods by treatment pre or post harvest of the materials themselves or precursor materials.

In addition to treating a host plant, seeds intended for consumption also can be treated using the subject formulations. The seeds can be dusted with a powder preparation (*see* U.S. Patent Application No. 4,978,686 for examples of inorganic materials to which the formulations can be adsorbed).

The invention further provides various "detoxifying" microorganisms that can be used to control the growth of toxin producing microorganisms or used to detoxify toxic metabolites present in a material. For example, fungal

growth can be controlled by inoculating the material, such as a plant or plant product, with one or more species of bacteria which reduce the growth of toxin producing bacteria and or fungi. Other microorganisms also can be used for inoculation which degrade or convert a toxin into a less or non-toxic form. Examples of microorganisms suited for inoculation include *Bacillus megaterium, B. lichenformis, B. subtilis, Cryptococcoses.* Of course other detoxifying bacteria can be used to achieve essentially the same result. For example, other detoxifying bacteria may be identified using standard screening methods where a toxin producing bacteria and or fungi is co-cultured with one or more other bacteria to be screened for detoxifying activity. The above-described anti-fungal compositions also can be used separately or in conjunction with these and other bacteria.

The target pathogenic organisms include fungi which colonize a surface of a part of a plant which is an elicitor for the fungus. By elicitor is intended that the plant provides nutrients required by the fungus. Of particular interest are those fungi which produce fumonisins and fusaric acid and their structural analogs. In order to determine the susceptibility of particular fungi to the claimed compositions, *in vitro* and *in vivo* tests such as are described in the Examples can be used and the change in the infestation rating (pre and post treatment) calculated as the MPDC.

The invention further provides methods and compositions for the identification and quantitation of various microbial toxins and toxin producing microorganisms present in a sample under field conditions, and or material obtained from the field, and or materials derived therefrom. Most preferably, the assays are used to test a variety of plants and agricultural products derived therefrom, including food stuffs and animal feed. If a material is contaminated with unacceptable levels of toxin, depending on the level of contamination and toxicity of the microbial toxin detected, the sample may be disposed of and or detoxified. Samples to be evaluated may be collected and prepared using standard techniques known in the art which are suited for a given assay.

In particular, the invention provides assays that can be used to determine the level of microbial toxin contamination in a sample by assaying for the presence or absence of one or more toxin producing fungi and or bacteria which are capable of colonizing a material of interest. Preferably, the assay is used to detect microorganisms which produce toxic metabolites, and particularly fungi and bacteria which produce secondary metabolites that are toxic to plants and or animal. Examples of fungi include those from the genera *Ceratucystis, Fusicoccum, Helminthosporium, Rhynochosporium* and *Stemphyllium* and most preferably those from the genera *Aspergillus, Alternaria, Fusarium,* and *Penicillium.* Examples of bacteria include *Corynebacterium, Erwinia, Pseudomonas* and *Xanthomonas.* The assay may also be used to detect the presence or absence of one or more non-toxin producing microorganisms which is indicative of the presence or absence of one or more toxin producing microorganisms. Examples of non-toxin producing microorganism or bacteria suitable for use in the assay include *Bacillus megaterium, Bacillus lichenformis, Bacillus subtilis,* and *Cryptococcoses.*

Assays used to identify microorganisms are known in the art. These methods include the use of chromatography techniques and subsets thereof which employ lipid, protein and/or nucleic acid identification methods. For example, recombinant DNA technology techniques may be used which employ the polymerase chain reaction and/or detectable probes. Nucleic acid, antibody, and or chemical probes may be used, for example, to directly or indirectly detect one or more microorganisms of interest. Other methods include microscopy and phenotypic evaluation of the plant or material tested. Microbiological screening techniques may also be used. For example, assays which rely on microorganism culturing may be employed where certain test microorganisms are capable of detectably responding to the presence or absence of one or more target microorganisms.

In another embodiment, assays are provided to determine the level of microbial toxin contamination by assaying for the presence or absence of one or more microbial toxins. Preferably, the assay is used to detect one or more toxic metabolites produced by one or more microorganisms that are capable of colonizing a material of interest. Microbial toxins of interest include bacterial toxins and toxic metabolites produced by fungi. More particularly, the assay is used to detect one or more secondary metabolites produced by fungi that are toxic to plants and or animals. Most preferably, the assays are used to test for the presence or absence of fungal mycotoxins referred to as aflatoxins, fusaric acid, fumonisins, TA/AAL toxins, aflatoxins, zearalenone, trichothecene, 5-butylpicolinic acid and related phytotoxic pyridine derivatives.

The assays of the present invention include the use of detectable probes, for example, various chemical and antibody probes, which react with the toxin of interest or some other material produced by one or more toxin or non-toxin producing microorganisms which is indicative of the presence or absence of toxin. The probes may either directly or indirectly detected using standard techniques. Most preferably, the assays include detectable chemical and or antibody probes. Microbial sensitivity tests may also be performed to test for the presence or absence of one or more microbial toxins, or non-toxins indicative of the presence or absence of one or more microbial toxins, using methods well known in the art. For example, the sensitivity of certain microorganisms to the presence of microbial toxin may be determined using a standard Ames test.

In another embodiment, the presence or absence of toxin-producing microorganisms, or the microbial toxins themselves, can be assayed using a test animal which detectably responds when exposed to toxin and or the toxin producing microorganism. In a preferred embodiment, a test animal is exposed to a sample containing various levels of one

or more microbial toxins and or toxin producing microorganism. The animals may be exposed to toxin material as single agents or in combinations. Control animals exposed to placebo samples are preferably included in the test.

In another embodiment, an assay is provided which is used to test the toxic effects that one or more microbial toxins or toxin producing microorganisms have on a test animal. In particular, these methods may be used to assess the role of toxin in cancers suspected of arising in part as a result of mycotoxin exposure, most specifically oral, esophageal, and lung cancers. Toxicity will depend on factors such as the level of toxin ingested by the test animal, the type of animal tested and the length of a study. Toxin or toxin producing microorganism may be provided to a test animal in food, water, air or by general environmental exposure. Toxicity and tolerance levels will depend on the body weight and the maturity of the animal. It is expected that most of these levels can be set with specificity after clinical testing.

Risk exposure levels for various mycotoxins will vary depending on the use of the feed, food or tobacco. Different animals have different tolerances. Also the effects of the toxicity may be expected to appear only beyond the life expectancy of the animal or person. Adverse effects of contact with mycotoxins is minimized among field workers, researchers and test animals. In this way, the invention can be used to generally improve agricultural products so that they are safer for human, animal, fish or fowl consumption. By controlling microbial toxin levels associated with plants and products derived therefrom, animal and human health problems associated with the consumption of toxin may be reduced or eliminated.

The invention also finds use for reducing the adverse effects of chewing and smoking tobacco, as it is a theory of the invention that many tobacco-related diseases may result from the presence of mycotoxins in tobacco products. Thus, tobacco toxicity can be reduced by adding substances to reduce mycotoxins and other secondary metabolites, in particular by treating the tobacco with sufficient amounts of a pathogen-inhibiting composition to inhibit fungal metabolite production.

Similarly, the invention can be used to decrease mycotoxin contamination of evergreen and fruit trees, gross lumber, grapes, ornamental, grasses and contamination resulting from grafting of fruit trees. Further, prevention of such contamination allows plant derived foodstuffs or by-products materials to be disposed of in a manner that does not release toxins in a form that can become waterborne or airborne. Thus, the invention can be used to assist in the safe disposal of agricultural by-products such as rice, wheat and cotton hulls and plant debris in general, which are often disposed of by burning. Furthermore, the toxic contents of products made from plant materials known to be colonized by toxin producing microorganisms can be improved by determining the level of secondary metabolites produced by particular species of fungi, which colonize materials pre-harvest, for example, *Fusarium* species, and/or post-harvest, for example, *Aspergillus* species. In particular, corn products like high fructose corn syrup can be improved by determining the level of certain toxins produced by *Fusarium* and then taking steps as necessary to reduce such levels.

Toxic secondary metabolites identified from organisms such as *Fusarium* spp. may also find use as pharmacological agents. It is to be anticipated that in this area, the recognition of previously unappreciated chemical structures will yield a number of structures for research as to their properties as herbicides, insecticides, fungicides, and pharmacological agents and for research into methods for producing the structures in commercial amounts.

The following examples are offered by way of illustration and not by way of limitation.

## EXAMPLES

### Example 1

### Treatment of Fungal Pathogens on Corn

A three-treatment experiment with cinnamic aldehyde formula and components, coniferyl aldehyde formula and combined cinnamic and coniferyl aldehyde formulas is evaluated on field grown corn known to be susceptible to pathogenic fungal infestation. The plants are blocked by variety before fungicide treatments and are randomized as to the plants. Various varieties susceptible to fungal infestation are tested using the following protocol which evaluates the effect of cinnamic aldehyde and/or coniferyl aldehyde alone and in combination with Tween 80 and/or $NaHCO_3$.

Each plant receives a single foliar spray to run off following evaluation of fungal infection (after Paulus and Nelson (1988) *Calif. Agric.* 42:15). The response variable recorded for each plant is the fungal infection rating based on the Paulus/Nelson (*supra*) rating scale. Plants are evaluated on this scale just prior to and four days after treatment.

Example 2

Treatment of Pitch Canker Disease
(*Fusarium subglutinans*)

(a) *In vitro* test

Pitch canker disease, caused by the fungus *Fusarium subglutinans* f.sp. pini is characterized by a resinous exudation on the surface of shoots, branches, exposed roots and boles of infested trees. The host and geographic range of the pitch canker pathogen has greatly increased since it was first discovered in California in 1986. The pathogen has recently been discovered in Mexico and Japan. An association of Engraver beetles (Scolytidae; IPS species) as vectors of the Pitch Canker Fungus has been proposed by Fox *et al.* (1991) *Plant Dis.* 75:676-682).

A bioassay based on inhibition of radial growth of *Fusarium subglutinans* f. sp. pini was used to test various aldehyde formulations. The bioassay was performed in a double blind fashion under sterile conditions. All concentrations given are those of particular solutions before dilution in the agar. Eight ml of test formulation was pipetted into 200 ml of molten 2% potato dextrose agar (DIFCO), and the mixture was dispersed into 5 plastic petri dishes (25 ml dish). Test formulations of 5 ppm benomyl were used as a positive control and sterile $H_2O$ as a negative control. Each of four plates was inoculated at the center with an agar plug transferred from a growing PDA culture of *Fusarium subglutinans* f. sp. pini (isolate SL-1, UCB) for each test formulation. A fifth plate was left noninoculated as a control for inhibited growth.

All inoculated and non-inoculated plates were incubated at 18° C for 5 days, after which colony diameters were measured. The results were provided in the Table below. The larger the colony, the less effective the test formulation. No growth of the colony (diameter=0) indicates a maximum growth inhibition.

The results show that concentrations of cinnamic aldehyde in vehicle (with or without the addition of saponin) inhibited radical growth of *Fusarium subglutinans,* as did 2% glutaraldehyde. Radial growth was completely inhibited at 12,500 ppm. (*See* Table 1.) The increase in radial growth seen with the addition of saponin may be due to the growth promotant characteristics which have been reported for saponin.

(b) Treatment of Monterey Pine Seeds

Seeds of Monterey Pine, half of which are dusted with a starch-dexta formulation containing cinnamic aldehyde and/or coniferyl aldehyde, 10 to 1000 ppm, are planted in vermiculite into which has been admixed a formulation of cinnamic aldehyde and/or coniferyl aldehyde, 10 to 1000 ppm. An inoculum of *Fusarium* which causes pine canker disease is added to the vermiculite at the time the seeds are planted. Seed in untreated vermiculite are used as a control.

Example 3

Treatment of Strawberry Red Core
(*Phytophthora Fragariae*)

Strawberry red core disease is caused by the fungus *Phyytophthora fragariae* Hickman which is spread by means of infected planting material or soil infested with long-lived oospores of infected debris. Various formulations containing cinnamic aldehyde and/or coniferyl aldehyde are tested as follows: Macerated strawberry roots infected with *Phytophthora fragariae* are thoroughly mixed with infested compost and allowed to decompose for 4 to 6 weeks to produce a well rotted inoculum for treatment. This is divided into 1 kg lots and mixed with 1500 ml of a test formulation at different concentrations (*see* Table 2). After 10 minutes treatment, the compost is rinsed under running tap water on a 25 mm sieve for a minimum of 5 minutes to remove all traces of the test formulation. The compost is then put into 9-cm diameter plastic pots and planted with 4 strawberry plants per pot. Five pots are used for each treatment. Plants are grown in a controlled environment room at 15°C and 18 h daylength; the compost is kept damp to encourage infection. Pots are placed on grids to avoid cross infection among treatments.

After 9 weeks the strawberry plant roots are washed free of compost and examined for signs of infection by cutting roots longitudinally and looking for red steles, and rotted or brown roots. All infections are confirmed by microscope examination of root pieces for the presence of oospores of *Phytophtora fragariae.*

Table 1

| Effect of Aldehydes on Radical Growth of *Fusarium subglutinas f.sp. pini* | | | | | | | |
|---|---|---|---|---|---|---|---|
| | Cinnamic Aldehyde[1] (ppm)[2] | | | | | | |
| | None | 10 | 100 | 2,500 | 5000 | 12,500 | 25,000 |
| None | 4.038 | 4.363 | 4.238 | 3.513 | 2.980 | 0 | 0 |
| Saponin[3] | 4.138 | 4.088 | 4.300 | 3.600 | 2.913 | 0 | 0 |
| Benomyl[4] | 0 | | | | | | |
| Tween 80(2%) + $NaHCO_3$(6%) | 4.380 | | | | | | |
| Glutaraldehyde[5] | 3.663 | | | | | | |
| $H_2o$ | 3.738 | | | | | | |

[1] All cinnamic aldehyde formulations are made up in 2% Tween 80, 6% $NaHCO_3$

Table 2

| Treatment Protocol | | | |
|---|---|---|---|
| Group | Treatment | Ingredient(s) | Amount of treatment ingredient(s) (balance $H_2O$ to 1000g |
| 1 | A | Cinnamic aldehyde | 5g |
| 1 | B | Tween 80 (T80) | 10g |
| 1 | C | $NaHCO_3$ | 80g |
| 1 | D | Cinnamic aldehyde + T80 | 5g, 10g |
| 1 | E | Cinnamic aldehyde + $NaHCO_2$ | 5g, 80g |
| 1 | F | $NaHCO_3$ + T80 | 80g, 10g |
| 1 | G | Test formulation 1 | A=5, B=10g, C=80g |
| 1,2,3 | H | +Control (Benomyl) | per manufacture instructions |
| 1,2,3 | I | -Control | no treatment |
| 2 | J | Coniferyl aldehyde (COFA) | 5g |
| 2 | K | COFA + T80 | 5g, 10g |
| 2 | L | COFA + $NaHCO_3$ | 5g, 80g |
| 2 | M | Formula 2 | J=5g, B=10g, C=80g |
| 3 | N | Cinnamic aldehyde + COFA | 2.5g, 2.5g |
| 3 | O | Cinnamic aldehyde+COFA+T80 | 2.5g, 2.5g, 10g |
| 3 | P | Cinnamic aldehyde+COFA+ $NaHco_3$ | 2.5g, 2.5g, 80g |
| 3 | Q | Formula 3 | A=2.5g, J=2.5g, B=10g, C=80g |

Example 4

Post-Harvest Handling of Citrus Fruit

The purpose of this experiment is to evaluate the citrus fruit post-harvest preservative activity of cinnamic

aldehyde. Forty California oranges are selected at random from a packing house lot after elimination of freeze-damaged fruit. Twenty treatment oranges are treated in a tank with soap and concentration of test formulation, then washed and brushed with soap and biocide. After 5 minutes, treated citrus are rinsed in fresh water. Controls are only washed with fresh water. Both treated and untreated lots are dried (water eliminated from fruit surface). The treated lot is sprayed in tumbler with 100 ml of formulation, air dried 10 minutes, packed in carton and placed in temporary storage. The control lot is sprayed in tumbler with 100 ml of distilled $H_2O$, air dried 10 minutes, packed in carton and placed in temporary storage. After twenty days both lots are evaluated for physiological and pathological breakdown.

Example 5

Post-Harvest Handling of Underground Vegetables (Roots, Tubers and Bulbs)

The purpose of this experiment is to evaluate the underground vegetable preservative activity of cinnamic aldehyde. The edible portions of this group of vegetables develop mostly underground and include several botanical structures.

- Roots: beet, carrot, celeriac, radish, horseradish, parsnip, sweet potato, cassava, jicama;
- Tubers: potato, Jerusalem artichoke, yam;
- Bulbs: onion, garlic, shallot.

Method

Forty tuber potatoes are selected at random from field bins. Potatoes are cured in a packinghouse for six days to insure formation of periderm. Potatoes are removed from curing and into two groups of 20 each for trial. Twenty potatoes in treated group are washed, then sprayed with 10 ml each of formulation. Untreated potatoes are washed with clean $H_2O$. Treated and untreated potatoes are packed separately in consumer package and put in temporary storage. Potatoes are observed and evaluated at intervals of 30, 60, 90 and 120 days for pathological breakdown.

Example 6

Post-Harvest Methods for Fruit Vegetable Handling

The aim of this experiment is to evaluate the fruit vegetable preservative activity of cinnamic aldehyde. Fruit vegetables are not generally adaptable to long-term storage. Exceptions are hard-rind (winter) squashes and pumpkin. Immature fruit vegetables of concern are the legumes. curcurbits (soft rind squash) solanaceous vegetables (eggplant, peppers, *etc*.), okra and sweet corn. Mature fruit vegetables of interest are curcurbits (cantaloupe, honeydew or other muskmelons: watermelon, hard-rind squashes and pumpkins). (Solanaceous vegetables: mature green and vine ripe tomatoes, ripe peppers.)

Method

Forty fruit vegetable tomatoes (vine ripe) are selected at random from bulk gondolas at a packing house. Twenty treated tomatoes are clear water rinsed, then treated with spray of 100 ml of test formulation on roller conveyer section. Twenty untreated tomatoes are sprayed with 100 ml $H_2O$ on roller conveyor sections. Treated and untreated are put in separate place pack (treated and untreated) and stored in temporary storage for 10 days. Tomatoes are observed and evaluated on days 3. 6 and 10 for pathological breakdown.

Example 7

Preservative Activity of Cinnamic Aldehyde

The aim of this experiment is to evaluate the flower, leafy and stem vegetable preservative activity of cinnamic aldehyde. The leafy, stem and floral vegetables are represented by (not limited to) the following commodities:

- Leafy vegetables: lettuce, cabbage, Brussel sprouts, celery, spinach, green onions, Witloaf chicory, endive;
- Stem vegetables: asparagus, kohlrabi, fennel:
- Floral vegetables: artichoke, broccoli, cauliflower

Method (Post-Harvest Example of Floral Vegetable: Cauliflower)

Forty heads of cauliflower are selected from a packinghouse lot. Leaves are trimmed from cauliflower. Twenty treated heads are sprayed with 10 ml test formulation each and the heads wrapped. Twenty untreated heads are sprayed with 10 ml $H_2O$ and then wrapped. Treated and untreated heads are put in separate boxes and stored in temporary storage for 10 days. Heads are observed on days 3, 6 and 10 for pathological breakdown.

Example 8

Post-Harvest Treatment of Cut Flowers

Rose Water Solution Microbes

Long life of cut flowers depends on maintenance of sufficient water status of the cut flowers from the moment of harvest until the flower fades in the home. The two critical factors to good water status are rehydration after dry handling, and control of vase solution bacteria whenever the stems are in water. Although a range of commercial biocides are used in vase preservatives for cut flowers, none is particularly effective or long lasting. This bioassay tests the possibility of preventing the growth of bacteria in vase solutions by using a cinnamic aldehyde formulation which has the potential for a new type of vase preservative and cut flower vase solution biocide.

Methods

Twenty fresh decapitated roses (*Hybrid tea*) and twenty fresh decapitated carnations (*Dianthus caryophyllus*) are cut so that 4 cm of stem extends beyond the ring top of a plastic baby nursing bottle. Flowers are each placed in separate bottles secured to bench with tape and lined with sterile baby bottle liners. Ten of each type of cut flowers serve as treatment group, ten as control. Both flower groups are given 10 ml $H_2O$. Treated cut flowers receive range of formulation from 20-50 ppm. Senescence of cut flowers is observed and recorded.

Example 9

Overproduction of Aromatic Aldehydes in Transgenic Plants

Twenty micrograms of polyA RNA is prepared and cDNA synthesized. Part of this is cloned into lambda-ZAP II vector (a commercially available cloning vector). At least 500,000 recombinants are screened using an oligonucleotide probe designed from conserved sequences of cloned CA4H and CAD genes obtained from GenBank, or designed from peptide sequence of purified protein from the intended host plant. Strongly hybridizing clones are selected and used to rescreen the cDNA library. The resulting clones are sequenced to enable the introduction of appropriate gene sequences into a plant expression cassette in either antisense or sense orientation. The antisense and sense constructs are introduced into *Agrobacterium tumefaciens* LBA4404 by direct transformation following published procedures. Tobacco (*N. tabacum* var. Samsun) leaf discs are transformed using well established published procedures (Horsch *et al.* (1985) *Science* 227:1229-1231). Plants containing either CA4H or CAD constructs are identified by PCR and selected for further analysis.

Plant material from both transformed and untransformed control plants is used for determinations of CA4H and CAD enzyme activity using well established published assays. Plants in which the activity of CA4H or CAD has been reduced to less than 20% of that seen in control plants are selected for further analysis. Selected plants with low CA4H activity are crossed with plants with low CAD activity and progeny inheriting both gene constructs are selected by PCR. Plants with suppressed CA4H and suppressed CAD activity are analyzed for aromatic aldehyde production using standard published procedures.

Example 10

Production of aromatic aldehydes in microbial systems

A cDNA library is generated using RNA extracted from six week old tobacco stems. 20μg of polyA RNA is prepared and cDNA synthesized. Part of this is cloned into lambda-ZAP II vector (a commercially available cloning vector). At Least 500.000 recombinants are screened using an oligonucleotide probe designed from peptide sequence sequences of CCoAr protein purified from six week old tobacco stem tissue using the protocol of Goffner *et al.* (1994) *Plant Physiol.* 106:625-632. Strongly hybridizing clones are selected and used to rescreen the cDNA library. The resulting clones are sequenced to enable the identification of hall-length cDNA inserts and the introduction of appropriate CCoAR gene sequences into yeast expression vector pMTL8110 (Faulkner *et al.* (1994) *Gene* 143:13-20). The coding sequences for *Rhodosporidium toruloides* phenylalanine ammonia lyase (PAL; GenBank locus RHDPAL) and a parsley 4-coumarate:CoAI ligase (4CL: GenBank locus PC4CL1AA) are similarly introduced into equivalent yeast expression vectors. The PAL.4CL and CCoAR constructs are used to transform *Saccharomyces cerevisiae* strains by electroporation using established published procedures (Becker and Guarente (1991) *Methods in Enzymology* 194:182-187: Simon (1993) *Methods in Enzymol* 217:478-483). Transformants are selected on minimal medium lacking leucine. Transformant strains carrying all three gene constructs are identified by PCR and selected for further analysis.

Extracts from both transformed and untransformed control strains are used for determinations of PAL. 4CL and CCoAR enzyme activities using well established published assays. Strains in which the activity of PAL. 4CL and CCoAR is significantly greater than the background activity detected in control strains are selected for further analysis. Selected strains are analyzed for aromatic aldehyde production using standard published procedures and those producing significant amounts of cinnamaldehyde are selected for optimization of fermentation conditions.

Example 11

Treatment of Late Blight (*Phytophtora infestans*)

Late blight affects tomato, potato, eggplant and other potato family plants: it begins when fungal spores settle on wet plant surfaces during periods of mild temperature. Experiments to test for control of *Phytophtora infestans* are conducted using potato seedlings in the greenhouse. Plants are spray-inoculated with using an isolate of the pathogen in the greenhouse to achieve. Plants are treated either prior to or after the inoculation with the pathogen using a treatment protocol such as that shown in Table 2. including a third panel for testing alpha-hexyl cinnamic aldehyde. An additional treatment regimen using a sufficient amount of saponin in place of the Tween as an emulsifier is also tested. Treatment effects are evaluated at two and three weeks post-treatment.

As most food for human and animal consumption is of animal or plant origin, and given the known activity of microbial toxins, the presence of microbially produced toxins that accumulate before harvest and or after harvest and persist after processing are likely to be at least partially responsible for a multitude of health disorders linked to mycotoxin contamination. By controlling microbial toxin levels in consumable products and those found

As most food for human and animal consumption is of animal or plant origin, and given the known activity of microbial toxins, the presence of microbially produced toxins that accumulate before harvest and or after harvest and persist after processing are likely to be at least partially responsible for a multitude of health disorders linked to mycotoxin contamination. By controlling microbial toxin levels in consumable products and those found associated with certain materials found in the environment, cancer, liver disease and other diseases linked to microbial toxin ingestion may be reduced, particularly those associated with the consumption of contaminated cereal grain foods and animal feeds and those associated with processed grain products such as corn syrup. In addition, laboratory animal testing procedures may also be improved by controlling the mycotoxins associated with the animal foods and certain test materials such as tobacco. Moreover, by controlling the level of microbial toxin associated with tobacco, cancers in humans linked to tobacco chewing or smoking may be reduced.

Toxic metabolites associated with materials of interest include those produced by bacteria and or fungi, and especially fungal mycotoxins and other secondary metabolites having similar properties. The methods and compositions of the invention are particularly suited to control the level of one or more mycotoxins produced by fungi from the genera *Aspergillus, Alternaria, Fusarium,* and *Penicillium,* and more particularly those fungi which produce one or more mycotoxins including aflatoxins, fumonisins, fusaric acid, TA/AAL toxins, zearalenone, and trichothecene, 5-butylpicolinic acid and related phytotoxic pyridine derivatives.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

The invention now having been fully described, it will be apparent to one of ordinary skill in the art that many changes and modifications can be made thereto without departing from the spirit or scope of the appended claims.

**Claims**

1. A method for inducing resistance of plants to mycotoxin-producing microorganisms wherein at least one compound capable of inducing said resistance is produced in the plant of interest by modulating the expression of at least one gene of said plant, wherein said compound has a structure shown in formula (1):

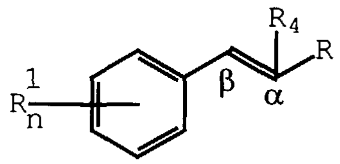

(1)

wherein

R represents $-CH_2OH$ or -CHO;
n is an integer from 0-3;
each $R^1$ independently represents OH or an organic substituent containing from 1-10 carbon atoms and from 0-5 heteroatoms, wherein the total number of carbon and heteroatoms in all $R^1$ substituents of said compound is no more than 15; and
$R_4$ represents hydrogen or an organic substituent containing from 1 to 10 carbon atoms,

wherein said compound is non-phytotoxic to said plant.

**2.** Method according to claim 1, in which also a saponin or a derivative thereof is produced in the plant of interest by modulating the expression of at least one other gene of said plant.

**3.** Method according to any of the previous claims in which said compound has a structure shown in formula (2):

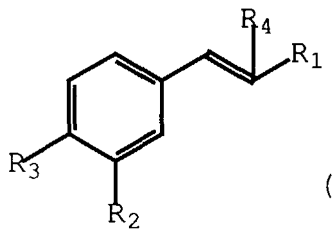

(2)

wherein

$R_1$ represents -CHO;
$R_2$ represents hydrogen, a methoxy group or an organic substituent containing from 1 to 10 carbon atoms;
$R_3$ represents hydrogen, OH or an organic substituent containing from 1 to 10 carbon atoms; and
$R_4$ represents hydrogen or an organic substituent containing from 1 to 10 carbon atoms.

**4.** Method according to any of the previous claims in which said compound is cinnamic aldehyde, coniferyl aldehyde and/or $\alpha$-hexyl cinnamic aldehyde.

**5.** Method according to any of the previous claims in which said modulating comprises recombinant DNA techniques.

**6.** Method according to any of the previous claims in which at least one compound is expressed by the plant and at least one other compound is applied exogenously.

**7.** Method according to any of the previous claims in which the expression of cinnamate 4-hydroxylase and cinnamic alcohol dehydrogenase is downregulated.

**8.** Method according to claim 7 in which DNA constructs comprising at least 50 bases are used which are homologous to the cinnamate 4-hydroxylase and cinnamic alcohol dehydrogenase genes.

**9.** Method according to claim 7 or 8 in which the cinnamate 4-hydroxylase and cinnamic alcohol dehydrogenase genes or homologous constructs thereof are introduced separately into different plants followed by standard sexual cross to produce said plant.

European Patent Office

# EUROPEAN SEARCH REPORT

Application Number
EP 99 20 3496

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 94 08036 A (SALK INST FOR BIOLOGICAL STUDI ;UNIV OLDENBERG (DE); CLAUSEN MONIK) 14 April 1994 (1994-04-14)<br>* page 2, line 35-38 *<br>* page 3A, line 20 - page 4, line 14 * | 1-9 | A01N35/02<br>C12N15/63 |
| X | LAMB C J ET AL: "EMERGING STRATEGIES FOR ENHANCING CROP RESISTANCE TO MICROBIAL PATHOGENS"<br>BIO/TECHNOLOGY,US,NATURE PUBLISHING CO. NEW YORK,<br>vol. 10, 1 November 1992 (1992-11-01), pages 1436-1445, XP002032892<br>ISSN: 0733-222X<br>* page 1439, column 1, paragraphs 3,4 * | 1-9 | |
| D,Y | WO 93 05159 A (ICI PLC)<br>18 March 1993 (1993-03-18)<br>* page 3, line 20-31 *<br>* page 5, line 26-33 * | 1-9 | |
| P,Y | US 6 015 943 A (BOERJAN WOUT ET AL)<br>18 January 2000 (2000-01-18)<br>* column 2, line 28-60 *<br>* column 3, line 34-36 *<br>* column 16, line 54-65 * | 1-9 | TECHNICAL FIELDS SEARCHED (Int.Cl.7)<br>A01N<br>C12N |
| D,Y | US 4 978 686 A (SOTOME KIYOSHI)<br>18 December 1990 (1990-12-18)<br>* the whole document * | 1-9 | |
| Y | FR 2 529 755 A (SAOTOME KIYOSHI)<br>13 January 1984 (1984-01-13)<br>* the whole document * | 1-9 | |

The present search report has been drawn up for all claims

| Place of search | Date of completion of the search | Examiner |
|---|---|---|
| MUNICH | 23 March 2000 | Klaver, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 99 20 3496

This annex lists the patent family members relating to the patent documents cited in the above-mentioned European search report.
The members are as contained in the European Patent Office EDP file on
The European Patent Office is in no way liable for these particulars which are merely given for the purpose of information.

23-03-2000

| Patent document cited in search report | Publication date | Patent family member(s) | Publication date |
|---|---|---|---|
| WO 9408036 A | 14-04-1994 | NONE | |
| WO 9305159 A | 18-03-1993 | AU 669106 B | 30-05-1996 |
| | | AU 1658192 A | 05-04-1993 |
| | | BR 9205934 A | 05-07-1994 |
| | | CA 2109222 A | 27-10-1992 |
| | | EP 0584117 A | 02-03-1994 |
| | | JP 6509465 T | 27-10-1994 |
| | | US 5451514 A | 19-09-1995 |
| US 6015943 A | 18-01-2000 | FR 2718460 A | 13-10-1995 |
| | | AU 705401 B | 20-05-1999 |
| | | AU 2347295 A | 30-10-1995 |
| | | BR 9507291 A | 23-09-1997 |
| | | CA 2185334 A | 19-10-1995 |
| | | EP 0755449 A | 29-01-1997 |
| | | WO 9527790 A | 19-10-1995 |
| | | JP 9511647 T | 25-11-1997 |
| | | NZ 284823 A | 24-09-1998 |
| | | ZA 9502980 A | 11-01-1996 |
| US 4978686 A | 18-12-1990 | JP 2621114 B | 18-06-1997 |
| | | JP 63255203 A | 21-10-1988 |
| | | AU 609797 B | 09-05-1991 |
| | | AU 1447088 A | 13-10-1988 |
| | | BR 8801769 A | 20-12-1988 |
| FR 2529755 A | 13-01-1984 | BR 8206283 A | 17-04-1984 |

EPO FORM P0459

For more details about this annex : see Official Journal of the European Patent Office, No. 12/82